(19) **Europäisches Patentamt / European Patent Office / Office européen des brevets**

(11) **EP 4 417 235 B1**

(12) **FASCICULE DE BREVET EUROPEEN**

(45) Date de publication et mention de la délivrance du brevet:
**11.03.2026 Bulletin 2026/11**

(21) Numéro de dépôt: **24153432.0**

(22) Date de dépôt: **23.01.2024**

(51) Classification Internationale des Brevets (IPC):
***A61M 16/00*** *(2006.01)*

(52) Classification Coopérative des Brevets (CPC):
**A61M 16/1005; A61M 16/024; A61M 16/12; A61M 16/204; G16H 20/10; G16H 20/40; G16H 40/63;** A61M 16/0833; A61M 16/085; A61M 16/1065; A61M 16/16; A61M 16/22; A61M 2016/0039; A61M 2016/1035; A61M 2202/0208; (Cont.)

(54) **APPAREIL DE FOURNITURE DE GAZ, TEL DU NO, À UN PATIENT AVEC COMPTAGE DU TEMPS TOTAL DE TRAITEMENT EFFECTIF**

VORRICHTUNG ZUR BEREITSTELLUNG VON GAS, WIE NO, FÜR EINEN PATIENTEN, MIT ZÄHLUNG DER EFFEKTIVEN GESAMTEN BEHANDLUNGSZEIT

APPARATUS FOR SUPPLYING GAS, SUCH AS NO, TO A PATIENT WITH COUNTING OF THE EFFECTIVE TOTAL TREATMENT TIME

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorité: **20.02.2023 FR 2301524**

(43) Date de publication de la demande:
**21.08.2024 Bulletin 2024/34**

(73) Titulaire: **INOSYSTEMS**
**92160 Antony (FR)**

(72) Inventeurs:
• **BLANDIN, Yann**
**92160 Antony (FR)**
• **SCHMITT, Mary**
**92160 Antony (FR)**
• **MARCHAL, Frédéric**
**92160 Antony (FR)**

(74) Mandataire: **Air Liquide**
**L'Air Liquide S.A.**
**Direction de la Propriété Intellectuelle**
**75, Quai d'Orsay**
**75321 Paris Cedex 07 (FR)**

(56) Documents cités:
EP-A1- 3 701 989    EP-A1- 3 821 929
EP-A1- 3 865 165    WO-A1-2012/094008
WO-A2-02/40914     JP-A- H11 192 303
US-A1- 2003 116 159   US-A1- 2015 273 175
US-A1- 2015 320 951

(52) Classification Coopérative des Brevets (CPC):
(Cont.)A61M 2202/0275; A61M 2202/0283;
A61M 2205/3334; A61M 2205/505; A61M 2205/52;
A61M 2205/8206; F17C 2201/0109;
F17C 2201/0119; F17C 2201/032; F17C 2201/056;
F17C 2205/013; F17C 2270/025

(52) Classification Coopérative des Brevets (CPC):
(Cont.)A61M 2202/0275; A61M 2202/0283;
A61M 2205/3334; A61M 2205/505; A61M 2205/52;
A61M 2205/8206; F17C 2201/0109;
F17C 2201/0119; F17C 2201/032; F17C 2201/056;
F17C 2205/013; F17C 2270/025

**Description**

La présente invention concerne un appareil ou dispositif de fourniture de gaz thérapeutique, en particulier de monoxyde d'azote (NO), et par ailleurs une installation d'administration de gaz à un patient comprenant un tel appareil de fourniture de gaz thérapeutique. L'appareil inclut des moyens permettant de comptabiliser et consigner le temps de traitement effectif de tous les patients ayant été traités par administration de gaz au moyen de l'appareil et ce, depuis la première utilisation de l'appareil, en excluant les périodes de pause ou de non utilisation de l'appareil, par exemple entre deux patients consécutifs.

Le monoxyde d'azote inhalé ou NOi est un médicament gazeux couramment utilisé pour traiter des patients souffrant d'hypertension artérielle pulmonaire aiguë, en particulier les vasoconstrictions pulmonaires chez l'adulte ou l'enfant, y compris les nouveau-nés ou PPHN (en anglais pour *persistent pulmonary hypertension of the newborn* = hypertension pulmonaire persistante du nouveau-né), comme décrit par exemple par EP-A-560928 ou EP-A-1516639.

Une installation de mise en œuvre d'un traitement par NOi, couramment appelée installation d'administration de NO, comprend classiquement une ou des bouteilles de mélange NO/$N_2$ alimentant un dispositif d'administration et/ou de monitoring du traitement par NO, un ventilateur médical pour délivrer un gaz respiratoire, tel un mélange $O_2/N_2$ ou de l'air, auquel est ajouté le NO (i.e. NO/$N_2$) et un kit patient comprenant notamment une interface respiratoire, telle une sonde trachéale, et un ou plusieurs conduits flexibles.

Une telle installation d'administration de NO est utilisée en milieu hospitalier pour administrer le traitement par NOi et ainsi soigner les patients de l'hôpital ayant besoin d'inhaler du NO pour traiter leur hypertension artérielle pulmonaire.

Or, la posologie du traitement des patients varie d'un patient à l'autre, à savoir le dosage prescrit par le médecin, le mode en ventilation et du volume minute réglés sur ventilateur médical et de la durée de l'administration (sachant qu'elle peut être continue ou discontinue quand une reprise de l'administration de NO est nécessaire après une pause ou une tentative avortée de sevrage.

Dès lors, au sein des hôpitaux, il est très difficile de tracer l'utilisation faite de ce médicament par les équipes de soins et de suivre les données classiques de consommation.

Une problématique similaire peut exister par ailleurs pour d'autres gaz thérapeutiques, en particulier les mélanges équimolaires d'oxygène et protoxyde d'azote (MEOPA), i.e. les mélanges $O_2/N_2O$.

Or, connaître la durée de traitement donc d'utilisation réelle des appareils est nécessaire pour différentes raisons, comme des raisons médicales liées au traitement devant être suivi par le patient, des raisons de maintenance de l'appareil, des raisons comptables, notamment de facturation, des raisons de bonne gestion de l'usage des appareils dans une structure hospitalière, des raisons liées au suivi de la consommation de gaz, ou d'autres raisons.

Certains dispositifs de fourniture de gaz, en particulier de NO, intègrent un compteur qui se déclenche et commence à comptabiliser le temps de traitement dès détection d'un changement de position (i.e. fermé ou ouvert) du robinet commandant la sortie du gaz d'une bouteille de gaz sous pression, telle une bouteille contenant un mélange gazeux NO/$N_2$, qui alimente le dispositif de fourniture de gaz considéré. Cependant, un tel système n'est pas idéal car l'appareil ne sait pas reconnaître et prendre en compte les mises en pause du traitement par gaz ou encore les périodes de test ou analogues. Or, ces dernières peuvent durer de quelques minutes à plusieurs heures, ce qui implique que le compteur continue à comptabiliser ces durées de pause comme temps de traitement tant que le robinet n'est pas refermé par l'utilisateur. Le temps de traitement comptabilisé est donc forcément inexact et imprécis.

EP-A-3821929 propose de comptabiliser le nombre total de patients traités par administration de gaz thérapeutique, tel du NO, en réponse à l'appui par l'utilisateur sur une touche de l'appareil de fourniture de gaz lors du commencement d'une administration de gaz, puis à nouveau à la fin de l'administration de gaz. Ceci permet de connaître et d'afficher le nombre de patients ayant inhalé le gaz et la durée précise de traitement réel de chaque patient, i.e. traitement court ou long. Cependant, la durée totale d'utilisation de l'appareil pendant laquelle s'effectue une administration de gaz au patient n'est pas déterminée par l'appareil, ni par ailleurs affichée.

Parfois, il est demandé directement aux personnels médicaux, tels que médecins, infirmières ou autres, de consigner par écrit, typiquement sur une feuille de papier ou un cahier, toutes les données disponibles concernant le démarrage du traitement, tout ajustement de traitement (e.g. ventilation, posologie...) et toute mise en pause éventuelle ou tentative d'arrêt de traitement. Il convient ensuite de réaliser des calculs pour connaître la durée totale d'utilisation de l'appareil de fourniture de gaz. On comprend aisément que cette manière de procéder est source d'erreurs de transcription et lors des calculs, d'oublis, de perte des données écrites.... et n'est donc pas une solution adéquate.

Un problème est dès lors de pouvoir connaître précisément et automatiquement le temps de traitement réel pendant lequel l'appareil a fourni un gaz thérapeutique ayant servi à traiter effectivement un patient par inhalation dudit gaz thérapeutique, en particulier de NO, lequel temps de traitement réel soit consultable à tout moment par l'utilisateur, tel le personnel soignant, afin de fournir une information utile à l'utilisateur et ce, de manière instantanée, i.e. en temps (quasi-)réel, sans que le personnel soignant n'ait à opérer lui-même une consignation manuscrite, ni aucun calcul de durée.

La présente invention a pour objet un appareil de fourniture de gaz tel que défini dans la revendication 1, ainsi qu'une installation d'administration de gaz à un patient telle que définie dans la revendication 11. Des modes de réalisation avantageux de la présente invention font l'objet des revendications dépendantes.

Une solution de l'invention concerne alors un appareil ou dispositif de fourniture de gaz pour fournir ou délivrer un gaz thérapeutique à un patient, en particulier un gaz contenant du NO, tel un mélange NO/N$_2$, comprenant :

- au moins un passage interne pour fournir un flux de gaz, comprenant des moyens à valve,
- des moyens de pilotage (ou dispositif de pilotage) à microprocesseur (i.e. un ou plusieurs microprocesseurs) commandant les moyens à valve pour contrôler la fourniture de gaz, c'est-à-dire autoriser ou stopper/interdire la circulation du gaz dans ledit au moins un passage interne, et
- des moyens de sélection (ou dispositif de sélection), actionnables par un utilisateur, configurés pour permettre à l'utilisateur de débuter ou d'arrêter une fourniture de gaz.

Selon l'invention, les moyens de pilotage sont configurés pour comptabiliser une durée totale (D$_{tot}$) de fourniture effective de gaz par l'appareil correspondant au cumul (i.e. à la somme algébrique) de toutes les périodes de temps (dti) s'étant écoulées entre un début et un arrêt de fourniture de gaz par l'appareil, c'est-à-dire au fil de temps, aux patients successifs ayant reçu du gaz dans le cadre de leur traitement, i.e. à tous les patients ayant été traités successivement par administration de gaz en utilisant l'appareil.

Autrement dit, la durée totale (D$_{tot}$) de fourniture effective de gaz comptabilisée selon l'invention correspond à la somme ou cumul de toutes les périodes ou temps d'utilisation (dti) effective de l'appareil pour traiter réellement des patients, à partir de la première utilisation de l'appareil, c'est-à-dire dès le premier patient traité, à l'exclusion de toutes les périodes de non-fourniture de gaz au patient. Toutes les périodes de non-fourniture de gaz au patient comprennent les périodes de non-utilisation de l'appareil, comme entre deux patients successifs, et les périodes d'utilisation de l'appareil mais sans fournir de gaz destiné à traiter le patient, par exemple un temps de mise en pause de l'appareil pendant le traitement d'un patient donné, une opération de maintenance ou un test de bon fonctionnement, une démonstration de fonctionnement ou analogue.

La durée totale (D$_{tot}$) de fourniture effective de gaz est donc telle que :

$$D_{tot} = \Sigma \ dt(i) \quad avec : i \geq 1$$

où : i est le nombre de périodes dt d'utilisation réelle du gaz considérées, c'est-à-dire de l'ensemble des patients traités, donc typiquement i > 1.

La durée totale (D$_{tot}$) de fourniture effective de gaz est donc égale à un cumul (i.e. addition ou somme) de toutes les périodes d'utilisation réelle de l'appareil pour traiter des patient, et ce, depuis sa première utilisation (i.e. avec un premier patient) ou mise en service (c'est-à-dire le temps d'administration total et réel du gaz aux patients), mais exclut toutes les périodes de non-utilisation de l'appareil, en particulier la ou les périodes de pause ayant eu lieu pendant le traitement d'au moins un patient ou les durées de non-utilisation séparant deux utilisations successives pour deux patients consécutifs, ou autres. Ainsi, les périodes de maintenance de l'appareil pendant lesquels des fournitures de 'gaz-test' peuvent être opérées, ne sont pas comptabilisées non plus.

Il existe différentes manières d'exclure ces périodes de maintenance ou autres, comme par exemple exclure les durées inférieures à un seuil de durée minimale donnée (par ex. 10, 15 ou 20 min), ou celles s'écoulant après appui par l'opérateur sur une touche spécifique (par ex. lancement de test) ou après branchement sur l'appareil à tester/maintenir, d'un dispositif spécifique servant à la maintenance (par ex. une clé USB), ou autres.

Néanmoins, la plupart de ces périodes de non-utilisation ont une durée négligeable, i.e. de quelques minutes en général, par rapport aux durées d'utilisation normale et effective de l'appareil, c'est-à-dire pendant un traitement thérapeutique d'un patient avec administration de gaz, en particulier de NO inhalé, lesquelles durent typiquement plusieurs heures ou dizaines d'heures, notamment d'un à plusieurs jours.

Dès lors, comptabiliser (i.e. ne pas les exclure) les durées de maintenance/test et analogues n'est pas souhaitable mais ne fausserait pas ou de manière très négligeable et/ou non-représentative, le calcul de la durée totale (D$_{tot}$) de fourniture effective de gaz, en particulier sur de longues périodes, par exemple sur plusieurs semaines ou à fortiori sur plusieurs mois consécutifs.

Selon le mode de réalisation considéré, l'appareil ou dispositif de fourniture de gaz thérapeutique de l'invention peut comprendre l'une ou plusieurs des caractéristiques suivantes :

- il comprend en outre des moyens de sélection, actionnables par un utilisateur, configurés pour permettre à l'utilisateur d'opérer un choix parmi un début de fourniture de gaz et un arrêt de fourniture de gaz, c'est-à-dire pour débuter ou stopper/interrompre une fourniture de gaz.
- la durée totale (D$_{tot}$) de fourniture effective de gaz par l'appareil correspondant au cumul (i.e. somme algébrique) de toutes les périodes de temps (dti) s'étant écoulées entre un choix de début de fourniture de gaz (DF) et un choix d'arrêt de fourniture de gaz (AF) opérés par l'utilisateur par actionnement (e.g. appui digital ou autre mode de sélection) des moyens de sélection de l'appareil.

- ledit au moins un passage interne est configuré pour acheminer le flux de gaz entre (au moins) une entrée et (au moins) une sortie de gaz fournissant le gaz.

- il comprend plusieurs passages internes de gaz, notamment une ligne principale et une ligne de bipasse, équipés de moyens à valves.

- les moyens de sélection coopèrent avec les moyens de pilotage pour leur fournir au moins un signal de commande correspondant au choix opéré par l'utilisateur (i.e. début ou arrêt de fourniture de gaz).

- les moyens de pilotage (ou dispositif de pilotage) commandent les moyens à valve (ou dispositif à valve) pour contrôler le flux de gaz dans le passage interne, en réponse au signal de commande fourni par les moyens de sélection, de manière à autoriser ou stopper (i.e. interdire ou interrompre) toute circulation de gaz dans ledit passage interne.

- les moyens de pilotage sont configurés pour commander un affichage sur l'afficheur graphique de la durée totale ($D_{tot}$) de fourniture effective de gaz.

- les moyens de pilotage sont configurés pour commander un affichage sur l'afficheur graphique une ou plusieurs périodes de temps (dti) d'un ou plusieurs patients

- un algorithme comprend ou fait office (i.e. sert de) de compteur temporel, c'est-à-dire que le comptage du temps est opéré par un algorithme de comptage de durée.

- l'algorithme de comptage de durée, c'est-à-dire le compteur temporel, est mis en œuvre par les moyens de pilotage.

- le compteur temporel est configuré pour comptabiliser toutes les périodes de temps (dti) s'écoulant entre un choix de début de fourniture de gaz et un choix d'arrêt de fourniture de gaz opérés par l'utilisateur, en excluant toute période de non-fourniture de gaz (et préférentiellement les autres périodes susmentionnées : test, maintenance).

- le choix de début de fourniture de gaz correspond à un début de traitement chez un patient donné comprenant un commencement de fourniture de gaz ou à une reprise de traitement ayant été interrompu chez ce patient donné, en particulier en cas de mise en pause de la fourniture de gaz.

- le choix d'arrêt de fourniture de gaz correspond à un arrêt définitif de traitement dudit patient donné comprenant un arrêt définitif de fourniture de gaz à ce patient ou un arrêt temporaire d'un traitement ayant débuté chez un patient donné, en particulier lors d'une mise en pause de la fourniture de gaz audit patient.

- il comprend des moyens de mémorisation, aussi appelés dispositif de mémorisation, pour mémoriser la durée totale ($D_{tot}$) de fourniture effective de gaz et/ou toutes les périodes de temps (dti).

- le compteur temporel, aussi appelé compteur de temps, minuteur ou analogue, (i.e. *timer* en anglais) est intégré aux moyens de pilotage, en particulier à un microprocesseur des moyens de pilotage.

- le compteur temporel est configuré pour commencer à comptabiliser la durée totale ($D_{tot}$) de fourniture effective de gaz en réponse à un choix de début de fourniture de gaz opéré par l'utilisateur par actionnement des moyens de sélection, c'est-à-dire lors du démarrage d'un traitement du patient au début d'un traitement dudit patient ou lors d'une reprise (redémarrage) d'un traitement ayant été mise en pause.

- les moyens de pilotage sont configurés pour démarrer ou stopper, respectivement, le compteur temporel en réponse au signal de commande délivré par les moyens de sélection, en particulier un signal de début ou d'arrêt de fourniture de gaz, respectivement.

- le compteur de temps est configuré pour interrompre la comptabilisation de la durée totale ($D_{tot}$) de fourniture effective de gaz en réponse à un choix d'arrêt de fourniture de gaz, c'est-à-dire suite à un arrêt temporaire (i.e. mise en pause) ou définitif de traitement d'un patient.

- le compteur de temps est configuré pour ne pas comptabiliser de temps lors des périodes de non-utilisation de l'appareil, c'est-à-dire lorsque l'appareil ne fournit pas de gaz, en particulier toute période de pause et/ou toute période entre deux traitements successifs et/ou toute période de maintenance/test ou toute autre période de non-fourniture de gaz.

- la durée totale ($D_{tot}$) de fourniture effective de gaz comptabilisée par le compteur est mise à jour automatiquement dès démarrage du compteur, c'est-à-dire lors de chaque période de fourniture de gaz.

- le compteur est configuré (pour l'ensemble des patients traités successivement) :

  i) pour décompter (i.e. comptabiliser) la durée totale ($D_{tot}$) de fourniture de gaz uniquement lors d'une fourniture effective de gaz à un patient donné, c'est-à-dire lorsque l'appareil délivre du gaz destiné à ce patient, et
  ii) pour stopper tout décompte de durée totale ($D_{tot}$) dès interruption de fourniture effective de gaz à ce patient donné, et ce, que cette interruption soit définitive ou temporaire, par exemple durant le temps de remisage de l'appareil entre deux patients successifs, durant une mise en pause en cours de traitement d'un patient, durant une phase de test ou de calibration de l'appareil, durant une phase de démonstration... ou autres.

- le compteur est configuré pour répéter les étapes i) et ii) pour tous les patients successifs, c'est-à-dire traités successivement les uns après les autres au moyen de l'appareil avec fourniture effective de gaz à chacun desdits patients.

- le compteur temporel est configuré pour continuer à incrémenter, i.e. décompter ou comptabiliser, la durée totale ($D_{tot}$) de fourniture effective de gaz, au début de la fourniture de gaz à un patient suivant, sans remise à zéro (RAZ) dudit compteur temporel, après la fin de la fourniture effective de gaz à un patient précédent ayant été traité juste avant le patient suivant. Ainsi, si le premier patient a reçu du gaz pendant 9h 15min, le compteur temporel démarrera à 9h 15min (et non à 0h 0 min) lors du début de fourniture de gaz au patient suivant, c'est-à-dire au deuxième patient, et affichera alors 23h 45min par exemple en tant que durée totale ($D_{tot}$) de fourniture effective de gaz, à la fin de la fourniture de gaz au deuxième patient, si celui-ci a reçu du gaz pendant 14h30 (i.e. = 9h 15 min + 14h 30 min), et ainsi de suite avec les patients suivants.
- la durée totale ($D_{tot}$) de fourniture effective de gaz est exprimée en jours et/ou heures et/ou minutes.
- les moyens de pilotage coopèrent avec l'afficheur graphique pour afficher la durée totale ($D_{tot}$) de fourniture effective de gaz ayant été comptabilisée.
- les moyens de pilotage comprennent au moins un (micro)processeur, de préférence au moins un microcontrôleur.
- le (ou les) microprocesseur est agencé sur une (ou des) carte électronique.
- les moyens de pilotage comprennent au moins un microprocesseur (i.e. un ou des microprocesseurs) mettant en œuvre un ou plusieurs algorithmes, notamment au moins algorithme de calcul configuré pour permettre de déterminer la durée totale ($D_{tot}$) de fourniture effective de gaz.
- les moyens de sélection comprennent une ou des touches de sélection.
- la ou les touches de sélection sont une ou des touches virtuelles s'affichant sur l'écran tactile, c'est-à-dire une ou des touches tactiles.
- l'écran tactile est un écran interactif à dalle tactile permettant d'afficher des informations, des données ou autres et par ailleurs d'opérer des choix, sélections... par appui digital (doigt de l'utilisateur) ou à l'aide d'un stylet directement sur une zone de l'écran, notamment sur des touches virtuelles affichées par l'écran.
- l'afficheur graphique comprend un écran tactile et la ou les touches de sélection sont des touches virtuelles s'affichant sur l'écran tactile.
- l'afficheur graphique comprend un écran d'affichage digital, c'est-à-dire à dalle tactile. L'écran tactile comprend donc une dalle tactile.
- l'afficheur graphique est configuré pour afficher la ou les touches de sélection virtuelles sur l'écran d'affichage digital, sous forme d'un ou plusieurs zones, pavés ou fenêtres de choix ou analogue.
- l'afficheur graphique est configuré pour afficher en outre des informations, des mesures, des courbes, des alarmes, des graphiques ou tout autre donnée utile à l'utilisateur dans le cadre du traitement d'un patient.
- l'écran d'affichage en couleurs ou en noir et blanc, ou les deux. Par exemple, l'utilisateur pour choisir un affichage en couleurs ou en noir et blanc, selon sa préférence.
- l'afficheur graphique est relié électriquement aux moyens de pilotage de manière à pouvoir transmettre aux moyens de pilotage, les choix ou sélection opérés par l'utilisateur via les moyens de sélection, typiquement une ou des touches tactiles affichées sur l'écran d'affichage à dalle tactile.
- les moyens de pilotage sont configurés pour commander les moyens à valve pour opérer une fourniture de gaz en réponse à une sélection par l'utilisateur, i.e. un actionnement des moyens de sélection par l'utilisateur, d'un premier choix correspondant à un début de fourniture de gaz au patient, c'est-à-dire à un début de traitement du patient, ou à une reprise de fourniture de gaz (après mise en pause), c'est-à-dire à un re-démarrage de traitement du patient considéré.
- à l'inverse, les moyens de pilotage sont configurés pour commander les moyens à valve pour stopper toute fourniture de gaz en réponse à une sélection par l'utilisateur, i.e. à un actionnement des moyens de sélection par l'utilisateur, d'un second choix correspondant à un arrêt définitif de fourniture de gaz au patient, c'est-à-dire à un arrêt de traitement du patient, ou à une mise en pause de la fourniture de gaz, c'est-à-dire un arrêt temporaire, de l'envoi de gaz vers la patient, donc de son traitement par administration de gaz thérapeutique.
- il comprend une touche ou un bouton de sélection, de préférence une touche virtuelle, de « Début » de traitement commandant un début ou une reprise de fourniture de gaz à un patient donné.
- il comprend une touche ou un bouton de sélection, de préférence une touche virtuelle, d' « Arrêt » ou « Fin » de traitement commandant un arrêt temporaire ou définitif de fourniture de gaz audit patient considéré.
- la touche (ou bouton) de sélection de « Début » et la touche (ou bouton) de sélection d' « Arrêt » (ou « Fin ») de traitement sont une seule et même touche (ou bouton) commandant le début et la fin de la fourniture de gaz.
- alternativement, selon un autre mode de réalisation, la touche (ou bouton) de « Début » et la touche d' « Arrêt » (ou « Fin ») de traitement sont des touches (ou boutons) différentes, en particulier une première et une deuxième touche (ou bouton).
- les moyens de sélection peuvent aussi éventuellement comprennent une ou plusieurs autres touches (ou boutons) de sélection, comme par exemple :

  ▪ une touche de « Pause » permettant d'opérer une suspension ou arrêt temporaire du traitement, donc de la fourniture de gaz thérapeutique, après un début de fourniture de gaz, et/ou
  ▪ une touche de « Reprise» de traitement commandant une reprise ou un redémarrage de traitement avec fourniture de gaz à un patient donné, après une période de pause.

- les touches (ou boutons) de « Pause » et de « Reprise» de traitement sont une seule et même touche commandant la pause et la reprise de fourniture de gaz ou, de façon alternative, des touches (ou boutons) distinctes.

- il peut comprend en outre une touche (ou bouton) de confirmation, de préférence virtuelle s'affichant sur l'écran d'affichage, permettant de confirmer un choix opéré, par exemple de confirmer que le patient est le même patient ou un autre patient après arrêt de la fourniture de gaz, notamment pour distinguer entre un démarrage après un arrêt temporaire (i.e. une pause) dans le traitement d'un seul et même patient et après un arrêt définitif d'un traitement d'un patient donné et au moment du démarrage du traitement d'un nouveau patient.

- il comprend en outre des moyens de mémorisation d'informations, de données, de mesures, de calculs... ou autres, en particulier des moyens de mémorisation informatiques, i.e. numériques.

- les moyens de mémorisation sont configurés pour mémoriser (i.e. enregistrer) la durée totale de fourniture effective ($D_{tot}$) de gaz et/ou toutes les périodes de temps (dti).

- les moyens de mémorisation comprennent une ou plusieurs mémoires informatiques d'enregistrement et de stockage de données, par exemple une ou des mémoires volatiles ou non-volatiles, par exemple de type mémoire flash ou autre.

- au moins une mémoire (e.g. mémoire flash) est agencée sur une ou la carte électronique.

- il comprend en outre des moyens (i.e. un dispositif) d'alimentation en courant électrique servant à alimenter en courant électrique les composants de l'appareil de fourniture de gaz qui en requièrent pour fonctionner, tel qu'afficheur, microprocesseur(s), mémoire(s)... Les moyens d'alimentation en courant électrique peuvent comprendre, de manière classique, une (ou des) batterie rechargeable ou non, un cordon électrique et une prise d'alimentation connectable au secteur électrique (110/220 V), un transformateur de courant...

- il comprend en outre une carcasse, une coque ou un boitier rigide externe dans lequel sont agencés tout ou partie des composants du dispositif, en particulier les moyens de pilotage, le passage interne, les moyens à valve...

- l'afficheur graphique est agencé dans ou porté par une paroi de la carcasse rigide externe.

- la carcasse est en polymère.

- les moyens de pilotage sont configurés pour contrôler (au moins) les moyens à valve de manière à ajuster le débit de gaz thérapeutique circulant dans le passage interne.

- les moyens à valve comprennent une ou plusieurs électrovannes, de préférence plusieurs électrovannes agencées en parallèle.

- les moyens à valve comprennent au moins une (électro)vanne proportionnelle.

- il comprend en outre un dispositif débitmètre agencé sur le passage de gaz pour déterminer (i.e. mesurer) le débit de gaz thérapeutique circulant dans le passage interne.

- le dispositif débitmètre est du type à fil chaud ou à perte de charge.

- le dispositif débitmètre coopère avec les moyens de pilotage.

- il comprend en outre un dispositif régulateur de pression, tel un détendeur de gaz, agencé sur le passage de gaz pour contrôler, régler ou ajuster la pression de gaz thérapeutique au sein du passage interne.

- il comprend en outre des raccords et/ou connecteurs pour y raccorder fluidiquement des lignes de gaz, tels un ou des tuyaux flexibles amenant ou évacuant du gaz, ou y raccorder électriquement un ou des câbles électriques ou analogues.

- selon un mode de réalisation, les moyens de pilotage, en particulier le compteur temporel, sont aussi configurés pour déterminer, i.e. mesurer, la durée instantanée ($D_{inst}$) d'un traitement en cours d'un patient donné, c'est-à-dire le temps total de traitement de ce patient spécifique, en particulier un traitement par NO.

- l'écran d'affichage est configuré pour afficher la durée instantanée ($D_{inst}$), c'est-à-dire le temps total de traitement du patient en cours, en particulier un traitement par NO.

- les moyens de pilotage sont configurés pour commander un affichage de la durée instantanée ($D_{inst}$) sur l'afficheur graphique.

- il comprend des moyens de remise à zéro (RAZ) ou réinitialisation, de préférence une touche de réinitialisation, pour réinitialiser l'affichage de la durée instantanée ($D_{inst}$) sur l'afficheur graphique. Ce comptage et l'affichage de durée instantanée ($D_{inst}$) relatifs à un 'traitement en cours' sont remis à zéro quand un personnel soignant lance un démarrage d'un nouveau traitement, en cliquant sur une touche de « Début » de traitement ou de "Nouveau patient".

- les moyens de pilotage sont en outre configurés pour déterminer le nombre de traitements courts (TC) pour lesquels la durée totale de traitement est inférieure à une durée-seuil préfixée et/ou les durées respectives ($D_{TC}$) de ces traitements courts (TC). De préférence, on exclut de la comptabilisation des traitements courts (TC), les traitements ayant une durée minimale courte, c'est-à-dire un seuil-bas, correspond à une situation exceptionnelle ne correspondant pas à un traitement de patient, par exemple une correction d'erreur de réglage, un test ou autre, typiquement un seuil-bas inférieur ou égal à 30 min, de préférence inférieur ou égal à 20 min, typiquement inférieur ou égal à 10 minutes. Autrement dit, le nombre TC correspond au nombre de patients ayant été soumis à un traitement réel mais dont le traitement a été interrompu rapidement car ce traitement n'était pas efficace, engendrait des effets secondaires négatifs ou pour une autre raison.

- les moyens de pilotage sont en outre configurés pour déterminer le nombre de traitements longs (TL) pour lesquels la durée totale de traitement est supérieure à ladite durée-seuil préfixée et/ou les durées respectives ($D_{TL}$) de ces traitements longs (TL). Autrement dit, le nombre TL correspond au nombre de patients ayant été soumis à un traitement de longue durée car efficace chez ces patients, par exemple sur une durée pouvant atteindre plusieurs jours.

- typiquement, la durée-seuil préfixée est inférieure ou égale à 6 heures environ.

- la durée totale ($D_{tot}$) de fourniture effective de gaz par l'appareil correspondant donc aussi à la somme des durées ($D_{TL}$) des traitements longs (TL) et des durées ($D_{TC}$) des traitements courts (TC), à savoir : $D_{tot} = D_{TL} + D_{TC}$

L'invention concerne aussi une installation d'administration de gaz à un patient, en particulier d'administration d'un mélange gazeux contenant du NO, comprenant :

- au moins une source de gaz, en particulier de gaz thérapeutique à base de NO, tel mélange $NO/N_2$,
- un appareil de fourniture de gaz selon l'invention, alimenté en gaz par ladite au moins une source de gaz, et
- une ligne d'alimentation en gaz alimentée en gaz par l'appareil de fourniture de gaz selon l'invention.

Selon le mode de réalisation considéré, l'installation d'administration de gaz de l'invention peut comprendre l'une ou plusieurs des caractéristiques suivantes :

- elle comprend en outre un ventilateur médical, c'est-à-dire un appareil d'assistance ventilatoire, en communication fluidique avec la ligne d'alimentation en gaz.
- le ventilateur médical est relié fluidiquement à la ligne d'alimentation pour alimenter ladite ligne d'alimentation avec un gaz respiratoire contenant au moins 21% vol. d'oxygène, en particulier de l'air ou un mélange $N_2/O_2$.
- le ventilateur médical est un appareil d'assistance respiratoire fournissant le gaz à pression constante.
- alternativement, le ventilateur médical est un ventilateur de type HFO (High Frequency Oscillations) délivrant le gaz par oscillations à haute fréquence.

- la (ou les) source de gaz contient du NO gazeux, en particulier un mélange gazeux $NO/N_2$.

- la ligne d'alimentation en gaz est alimentée en un gaz thérapeutique, de préférence un mélange $NO/N_2$, par l'appareil de fourniture de gaz et en un gaz respiratoire contenant au moins 20% vol. d'oxygène, de préférence au moins 21%vol d'$O_2$, avantageusement de l'air ou un mélange $N_2/O_2$, par le ventilateur médical.

- la source de gaz contient un mélange $NO/N_2$ contenant moins de 2000 ppm en volume de NO, le reste étant de l'azote, de préférence moins de 1000 ppm en volume de NO, le reste étant de l'azote.

- préférentiellement, la source de gaz thérapeutique contient un mélange $NO/N_2$ contenant de 250 à 900 ppm en volume de NO, le reste étant de l'azote, par exemple de l'ordre de 800 ppm en volume de NO, le reste étant de l'azote.

- au moins une source de gaz thérapeutique contient un mélange $O_2/N_2O$, de préférence un mélange équimolaire (50%/50% mol) d'oxygène et de protoxyde d'azote, c'est-à-dire du MEOPA.

- elle comprend en outre un humidificateur de gaz agencé sur la ligne d'alimentation en gaz, de préférence en aval du site où le dispositif de fourniture de gaz thérapeutique est raccordé fluidiquement à ladite ligne d'alimentation en gaz de manière à l'alimenter en gaz thérapeutique.

- elle comprend en outre une ligne de récupération des gaz expirés par le patient.

- la ligne d'alimentation en gaz et la ligne de récupération des gaz expirés sont raccordées à une pièce de raccordement, de préférence une pièce en Y, et définissent un circuit patient.

- la ligne d'alimentation en gaz forme une branche inspiratoire du circuit patient.

- la ligne de récupération des gaz expirés forme une branche expiratoire du circuit patient.

- la ligne d'alimentation en gaz est raccordée fluidiquement à un port de sortie du ventilateur médical de manière à récupérer et acheminer le gaz délivré par le ventilateur médical.

- la ligne de récupération des gaz expirés est raccordée fluidiquement à un port d'entrée du ventilateur médical de manière à fournir au ventilateur médical tout ou partie des gaz expirés par le patient et à déterminer ainsi une ou des éventuelles fuites de gaz.

- la ligne de récupération des gaz expirés comprend éventuellement (i.e. non obligatoire) un dispositif d'élimination du $CO_2$ permettant d'éliminer le $CO_2$ présent dans les gaz expirés par le patient.

- la ligne de récupération des gaz expirés comprend éventuellement (i.e. non obligatoire) un filtre.

- au moins une source de gaz thérapeutique comprend un ou plusieurs récipients de gaz, en particulier une ou des bouteilles de gaz sous pression.

- le (ou les) récipient de gaz est équipé d'un robinet de distribution de gaz.

- le robinet de distribution de gaz est un robinet avec ou sans détenteur intégré (RDI).

- le robinet de distribution de gaz est en alliage de cuivre, tel du laiton.

- le (ou les) récipient de gaz est équipé d'un capotage de protection agencé autour du robinet de distribution de gaz.

- le capotage est en matériau polymère (i.e. plastique), en métal ou leurs combinaisons.

- le (ou les) récipient de fluide est une bouteille de gaz sous pression contenant, lorsqu'il est plein, un mélange gazeux, en particulier $NO/N_2$, à une pression d'au moins 150 à 200 bar abs, voire d'au moins 250 à 300 bar abs.

- le récipient de fluide a une forme générale cylindrique, en particulier d'ogive.

L'invention va maintenant être mieux comprise grâce à la description détaillée suivante, faite à titre illustratif mais non limitatif, en référence aux figures annexées parmi lesquelles :

Fig. 1 schématise un mode de réalisation d'un appareil de fourniture de gaz thérapeutique, en particulier de NO, selon l'invention,

Fig. 2 schématise un mode de réalisation d'une installation d'administration de gaz selon l'invention incluant l'appareil de Fig. 1,

Fig. 3 schématise un mode de réalisation d'un écran d'affichage de l'appareil de Fig. 1, et

Fig. 4 est une représentation graphique illustrative permettant de visualiser la durée totale (Dtot) comptabilisée selon l'invention.

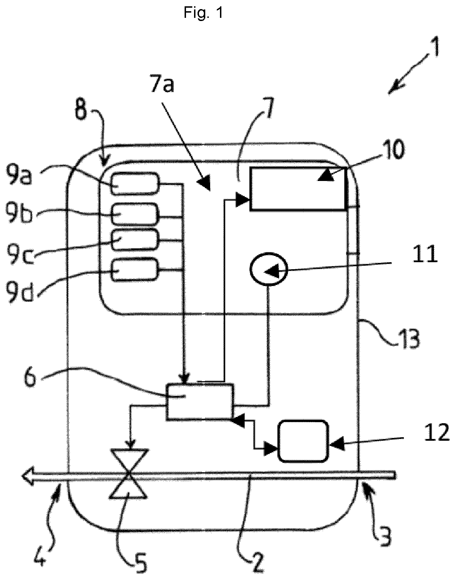

Fig. 1 schématise un mode de réalisation d'un appareil ou dispositif de fourniture de gaz thérapeutique 1 selon l'invention comprenant un boitier 13 rigide, par exemple en polymère, au sein duquel est agencé un passage de gaz interne 2, tel un conduit de gaz ou analogue, pour acheminer un flux de gaz thérapeutique.

Dans ce mode de réalisation, on prend comme exemple de gaz thérapeutique, un flux gazeux à base de NO, c'est-à-dire typiquement un mélange gazeux $NO/N_2$ formé de monoxyde d'azote (NO) et d'azote ($N_2$), permettant de traiter différentes pathologies pulmonaires, notamment des vasoconstrictions pulmonaires et/ou une hypertension pulmonaire, par exemple l'hypertension pulmonaire persistante des nouveau-nés ou PPHN.

Le flux gazeux à base de NO entre et circule dans le passage de gaz interne 2 entre une (ou des) entrée 3 et une (ou des) sortie 4 de gaz. Les entrée 3 et sortie 4 de gaz peuvent par exemple être portées par des connecteurs ou embouts de raccordement mécanique et fluidique portés par le boitier 13 à l'appareil 1, auxquels viennent se fixer des lignes de gaz servant à convoyer du gaz, comme expliqué ci-après, par exemples des tuyaux flexibles ou analogues.

Des moyens à valve 5, typiquement une ou plusieurs électrovannes, typiquement agencées en parallèle, sont agencés sur le passage de gaz interne 2 et permettent de contrôler le flux de gaz thérapeutique qui circule dans le passage interne 2, dans le sens allant de l'entrée 3 à la sortie 4 de gaz, par exemple une ou des (électro)vannes proportionnelles.

Optionnellement, un dispositif détendeur de gaz est agencé en amont des moyens à valve 5 pour réguler la pression du flux gazeux à base de NO au sein du passage de gaz interne 2, notamment pour garantir une pression donnée, par exemple de quelques bars.

Les moyens à valve 5 sont commandés par des moyens de pilotage 6 agencés dans le boitier 13, typiquement une carte électronique comprenant un (ou plusieurs) microprocesseur(s), typiquement un (ou des) microcontrôleur, mettant en œuvre un ou des algorithmes. Les moyens de pilotage 6 permettent notamment d'ajuster ou contrôler le débit de gaz traversant les moyens à valve 5.

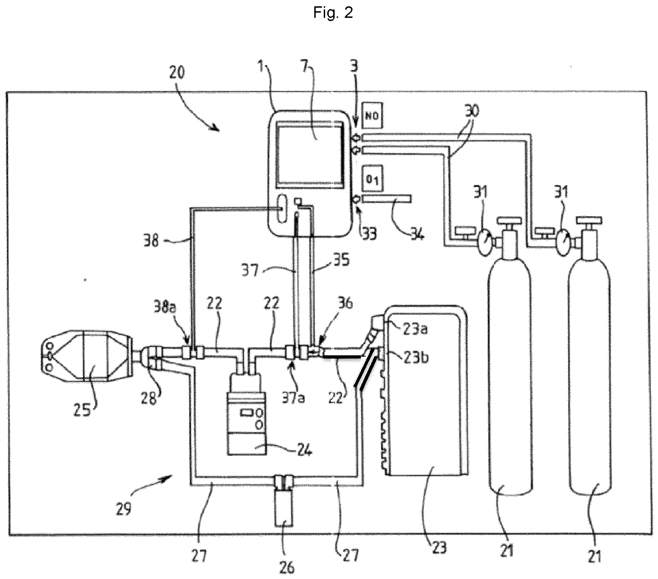

Ainsi, les moyens de pilotage 6 peuvent piloter les moyens à valve 5, telles des électrovannes agencées en parallèle, c'est-à-dire ouvrir ou fermer toutes ou partie de ces vannes, pour obtenir un débit de gaz (Q) déterminé/calculé par le microcontrôleur à partir d'une valeur réglée/fixée par exemple par l'utilisateur et en fonction du débit (Q') de gaz, i.e. air, délivré par le ventilateur 23 de Fig. 2, comme expliqué ci-après.

On prévoit aussi préférentiellement un (ou des) débitmètre (non représenté) agencé sur le passage de gaz interne 2, en amont et/ou en aval des moyens à valve 5 pour déterminer le débit de gaz (Q) à base de NO. Le débitmètre peut être du type à différentiel de pression, à fil chaud ou autre. Il coopère avec les moyens de pilotage 6 pour leur fournir des mesures.

Par ailleurs, le boitier 13 comprend un afficheur graphique 7, de préférence un écran tactile 7a, c'est-à-dire à dalle digitale, servant à afficher différentes informations ou données, en particulier différents choix sélectionnables par un utilisateur. Ces choix peuvent s'afficher dans des fenêtres ou analogues s'affichant sur l'écran tactile/digital 7a de l'afficheur graphique 7.

L'afficheur graphique 7 est aussi configuré pour visualiser une ou des courbes, graphiques, alarmes, icones.... ou toute autre information ou donnée utile à l'utilisateur, tel le personnel soignant.

Il est également prévu des moyens de sélection 8 permettant à l'utilisateur d'opérer une sélection parmi les choix sélectionnables qui sont affichés sur afficheur graphique 7, c'est-à-dire de choisir entre plusieurs options proposées et s'affichant à l'écran, ou de confirmer/valider ou de refuser un choix (i.e. une option), ou encore d'opérer des réglages ou ajustage, par exemple de sélectionner la valeur de débit souhaitée.

Les moyens de sélection 8 comprennent typiquement des touches ou boutons de sélection 9a-9d actionnables par l'utilisateur. Avantageusement, les moyens de sélection 8 comprennent des touches de sélection tactiles 9a-9d, c'est-à-dire des touches tactiles s'affichant sur l'afficheur graphique 7, typiquement un écran tactile.

Avantageusement, les moyens de sélection 8, typiquement des touches de sélection 9a-9d tactiles, permettent d'opérer un choix parmi plusieurs choix possibles. Par exemple, dans le mode de réalisation illustré :

- une touche tactile 9a de début de fourniture de gaz, c'est-à-dire de commencement du traitement d'un patient, i.e. une touche de « Début » de traitement,
- une touche tactile 9b d'arrêt de fourniture de gaz, c'est-à-dire d'arrêt définitif du traitement d'un patient, i.e. une touche d' « Arrêt » ou « Fin » de traitement.
- une touche tactile 9c de mise en pause de la fourniture de gaz, c'est-à-dire d'arrêt temporaire du traitement d'un patient, et de reprise de la fourniture de gaz après une pause, i.e. une touche de « Pause » et de « Reprise » de traitement.
- une touche tactile 9d de validation de choix (optionnelle), par exemple pour indiquer si le début de fourniture de gaz concerne un même patient (en cas de reprise après pause) ou, à l'inverse, un nouveau patient (après fin de traitement d'un premier patient).

Comme déjà indiqué, selon le mode de réalisation considéré, les touches tactile 9a de début de fourniture de gaz et 9b d'arrêt de fourniture de gaz peuvent être « fusionnée » en une seule et même touche commandant la fourniture et l'arrêt de fourniture de gaz, voire même être fusionnées aussi avec la touche tactile 9c pour commander aussi les pauses et reprises après pause. Selon le mode de réalisation désiré, certains choix différents peuvent donc être commandés par une seule et même touche, i.e. une touche commune unique, plutôt que par des touches différentes.

L'afficheur graphique 7, typiquement un écran à dalle tactile, est donc configuré pour permettre à un opérateur d'opérer les différents choix susmentionnés, i.e. début, pause, reprise et arrêt définitif de fourniture de gaz. Par exemple, en reprenant l'exemple ci-dessus, les touches 9a, 9b peuvent être « fusionnées » en une touche unique de démarrage/arrêt de la fourniture de gaz. De même, les touches 9c, 9d peuvent être « fusionnées » en une touche unique de mise en pause/redémarrage.

L'appui digital de l'utilisateur sur l'une des touches virtuelles 9a-9d affichées sur l'écran tactile 7a de l'afficheur 7, pour opérer l'un des choix susmentionnés va être transmis au microprocesseur(s) des moyens de pilotage 6 et celui-ci va piloter alors le fonctionnement de l'appareil (i.e. fourniture ou arrêt de fourniture de gaz) en fonction de la sélection opérée et, en parallèle, celui du compteur temporel, i.e. le démarrage ou l'arrêt du compteur temporel pour comptabiliser ou non le temps d'utilisation de l'appareil 1, comme expliqué ci-après.

Ainsi, les moyens de pilotage 6 peuvent être configurés pour commander les moyens à vannes 5, en réponse à l'appui sur l'une des touches virtuelles 9a-9d affichées sur l'écran tactile 7a de l'afficheur 7, donc à la réception du signal correspondant par lesdits moyens de pilotage 6, pour :

- autoriser une circulation de gaz dans le passage interne 2, au travers de la ou des électrovannes de contrôle de flux en fonction du débit (Q) fixé, en direction du patient en cas d'appui sur la touche 9a correspondant à un début de traitement par administration du gaz thérapeutique au patient, tel du NO, c'est-à-dire la touche 9a de « Début de traitement » ou sur la touche 9c de reprise après pause ;
- interrompre la circulation de gaz dans le passage interne 2 en direction du patient en cas d'appui sur la touche correspondant à un arrêt temporaire (pause) ou définitif du traitement chez le patient, c'est-à-dire la touche 9b de « Fin » de traitement ou celle 9c de mise en « Pause ».

La comptabilisation par les moyens de pilotage 6 de la durée totale d'administration active de gaz médical au patient se fait grâce au compteur temporel ou minuteur (i.e. *timer*), typiquement un compteur interne (e.g. intégré) du microprocesseur les moyens de pilotage 6 ou analogue.

Dit autrement, dès qu'un traitement par administration de gaz, tel du NO, doit débuter chez un patient, c'est-à-dire au début de la fourniture de gaz, l'utilisateur appuie sur la touche tactile « Début» de traitement 9a affichée sur l'afficheur graphique 7 correspond au lancement, i.e. début, de ce traitement, ce qui démarre la fourniture de gaz et la comptabilisation de la durée de traitement par le compteur temporel. Le compteur est interrompu à chaque pause ou à chaque arrêt de fourniture de gaz.

Selon l'invention, le décompte du temps n'a donc lieu que, lors des fournitures effectives de gaz, tel du NO, aux patients mais est interrompu dans toutes les situations dans lesquelles le gaz n'est pas fourni au patient, telles les pauses, les arrêts inter-patients, etc.... Les périodes de maintenance/tests ne sont pas non plus comptabilisées.

D'une façon générale, selon l'invention, les moyens de pilotage 6 sont configurés pour déterminer la durée totale ($D_{tot}$) de fourniture effective de gaz correspondant à la somme de toutes les périodes de temps dt(i) s'écoulant entre un choix de début de fourniture de gaz (e.g. début ou reprise d'un traitement) et un choix d'arrêt (e.g. pause ou définitif) de fourniture de gaz opérés par l'utilisateur.

Le compteur temporel s'incrémente donc progressivement au fil du temps, au fur et à mesure de l'utilisation de l'appareil 1. La durée totale ($D_{tot}$) de fourniture effective de gaz peut être exprimé en jours, heures et minutes, voire en semaines ou en mois.

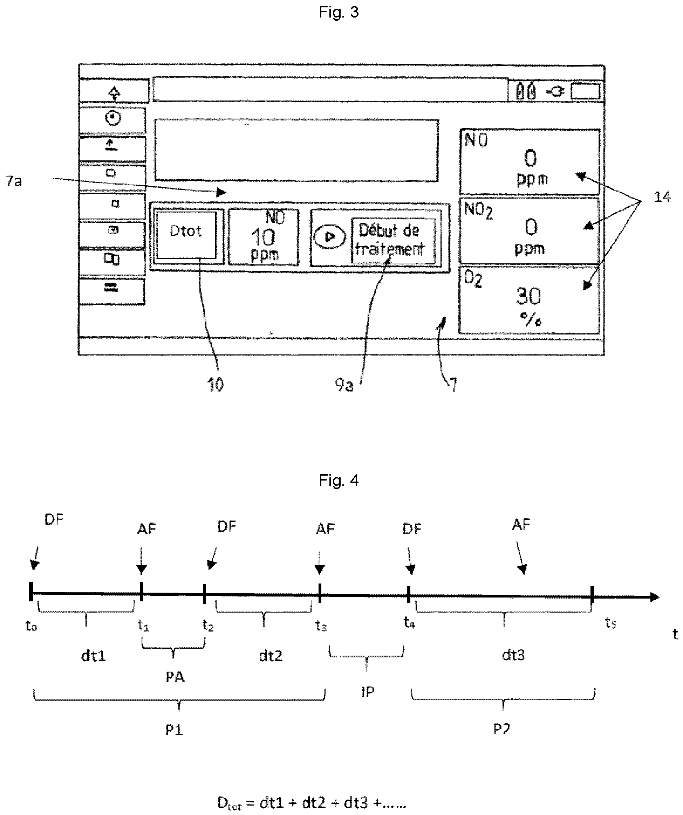

Autrement dit, comme illustré en Fig. 4, la durée totale ($D_{tot}$) de fourniture effective de gaz est telle que : $D_{tot} = \Sigma\ dt(i)$ avec : $i \geq 1$ où i est le nombre de périodes dt d'utilisation du gaz considérées.

Comme on le voit, le début de fourniture (DF) de gaz à un premier patient P1 commence à t0 et se poursuit pendant une première durée dt1 allant jusqu'à t1 où est opéré un arrêt de fourniture (AF) de gaz du fait d'une mise en pause PA de l'appareil 1, pendant un temps de pause débutant en t1 et se poursuivant jusqu'à t2.

A t2, la fourniture (DF) de gaz audit premier patient P1 reprend pendant une seconde durée dt2 avant d'être définitivement stoppée en t3 lorsque le personnel soignant considère que le traitement du premier patient P1 est terminé ou doit être stoppé.

Ensuite, l'appareil 1 n'est pas utilisé entre t3 et t4, qui est une période de temps inter-patient (IP), c'est-à-dire le temps séparant la fin du traitement du premier patient P1 et le début du traitement d'un second patient P2.

Le second patient P2 est alors traité pendant une période de temps dt3 s'écoulant entre t4 et t5, sans période de pause dans ce cas. Le traitement du second patient P2 est stoppé à t5 qui correspond alors à une nouvelle période de temps inter-patient (IP) précédant le traitement d'un troisième patient.

Selon l'invention, la durée totale ($D_{tot}$) de fourniture effective de gaz correspond donc au cumul, i.e. somme, des périodes de temps dt1, dt2, dt3.... dti.

La durée totale ($D_{tot}$) de fourniture effective de gaz depuis la première utilisation de l'appareil, c'est-à-dire sa première mise en service, est affichée sur l'afficheur graphique 7, soit en permanence, soit à la demande de l'utilisateur, par exemple après appui sur une touche de rappel dédiée ou autre.

Selon un mode de réalisation préféré, le compteur n'est jamais réinitialisé, c'est-à-dire remis à zéro (RAZ). Selon un autre mode de réalisation, le compteur peut être réinitialisé, c'est-à-dire remis à zéro (RAZ), après une période de temps longue donnée, par exemple après plusieurs mois ou année, ou après une maintenance ou autre.

Avantageusement, la durée totale ($D_{tot}$) de fourniture effective de gaz est affichée en permanence (en 10) sur l'écran de l'afficheur graphique 7 afin que le personnel soignant puisse en prendre connaissance à tout moment.

L'information de durée de fourniture effective de gaz ($D_{tot}$) peut être par ailleurs mémorisée dans une mémoire 12 de l'appareil, préférentiellement enregistré dans une mémoire informatique non-volatile.

Selon un mode de réalisation, l'information de durée de fourniture effective de gaz ($D_{tot}$), par exemple un nombre total d'heure et/ou de jours de traitement, peut être transmise à un système d'information hospitalier (SIH) ou Patient Data Management System (PDMS) en anglais. La connexion entre l'appareil 1 et le SIH peut être assurée par liaison filaire (i.e. câbles électriques), typiquement via des connexions RS232 ou HL7, ou par un réseau interne de l'hôpital, tel un intranet ou analogue, par exemple via un protocole de communication local, notamment en Wifi ou Bluetooth®. Ceci permet à l'hôpital de connaitre l'utilisation réelle de l'appareil 1, d'éditer des compte-rendu de soins médicaux ou autres.

Selon un autre mode de réalisation, l'information de durée de fourniture effective de gaz ($D_{tot}$) peut aussi être transmise par 4G vers à un serveur distant, notamment du fournisseur de l'appareil et/ou des gaz médicaux, tel le NO, de manière à lui permettre de suivre la « vie » de l'appareil 1 et de planifier les opérations de maintenance, de prévoir le remplacement de l'appareil (fin de vie d'utilisation légale), les réapprovisionnements en gaz ou autres, y compris d'établir des factures, des rapports d'utilisation...

D'une façon générale, le comptage des durées dt (i.e. dt1, dt2, dt3.... dti) d'administration active par le dispositif d'administration 1 de gaz de l'invention est géré par un algorithme faisant office de compteur temporel, i.e. minuteur, mis en œuvre par le microprocesseur des moyens de pilotage 6 du dispositif 1 et opérant comme expliqué ci-dessus.

Fig. 2 schématise un mode de réalisation d'une installation d'administration de gaz 20 selon l'invention, comprenant dans ce cas deux bouteilles 21 de gaz thérapeutique, à savoir ici des mélanges NO/$N_2$, par exemple contenant entre 200 et 1000 ppm vol. de NO (reste $N_2$), qui alimentent en mélange gazeux NO/$N_2$, un appareil 1 de fourniture de gaz selon l'invention, tel qu'illustré en Fig. 1 et décrit ci-avant.

Les bouteilles de gaz 21 sont reliées fluidiquement à l'appareil de fourniture de gaz 1 via des lignes d'amenée de gaz 30, tel que des tuyaux flexibles ou analogues, qui peuvent être équipées de dispositifs de régulation et/ou de suivi de la pression de gaz 31, tels que détendeur de gaz, manomètres ou autres. Les lignes d'amenée de gaz 30 sont reliées à une ou plusieurs entrées de gaz 3 du dispositif de fourniture de gaz 1 qui alimentent le passage interne 2 dudit dispositif de fourniture de gaz.

On note que le dispositif de fourniture de gaz 1 comprend aussi une entrée d'oxygène 33 reliée fluidiquement, via une ligne d'amenée d'oxygène 34, tel un tuyau flexible ou analogue, à une source d'oxygène (non montrée) par exemple le réseau hospitalier, c'est-à-dire une canalisation d'alimentation en oxygène agencée dans le bâtiment hospitalier, ou une bouteille d'oxygène sous pression.

Par ailleurs, est prévu aussi un ventilateur médical 23, c'est-à-dire un appareil d'assistance respiratoire, pour fournir de l'air ou un mélange oxygène/azote ($N_2$/$O_2$), c'est-à-dire un flux de gaz respiratoire contenant au moins 21% d'oxygène.

Le ventilateur médical 23 et le dispositif de fourniture de gaz 1 sont en communication fluidique avec une ligne d'alimentation en gaz 22 servant à acheminer le flux gazeux vers le patient.

Le dispositif de fourniture de gaz 1 délivre un mélange $NO/N_2$, par exemple 800 ppmv de NO, dans la ligne d'alimentation en gaz 22, via un conduit d'injection 37, de manière à injecter (en 37a) un flux de $NO/N_2$ dans le flux d'air ou de mélange oxygène/azote délivré par le ventilateur médical 23 et véhiculé par la ligne d'alimentation en gaz 22.

La ligne d'alimentation en gaz 22 comprend en outre un humidificateur de gaz 24 agencé en aval du site (37a) où le dispositif de fourniture 1 de gaz thérapeutique est raccordé fluidiquement à la ligne d'alimentation 22. Cet humidificateur de gaz 24 permet d'humidifier le flux de gaz, e.g. mélange $NO/N_2$/air, avant qu'il ne soit inhalé par le patient, au moyen d'une interface respiratoire patient, telle une sonde trachéale ou analogue. Sur la Fig. 2, l'ensemble patient/interface est schématisé par un « poumon artificiel » 25.

Est prévue aussi une ligne de récupération 27 des gaz expirés par le patient. La ligne d'alimentation en gaz 22 et la ligne de récupération 27 des gaz expirés sont raccordées à une pièce de raccordement 28, de préférence une pièce en Y, et définissent ainsi un circuit patient 29. La ligne d'alimentation en gaz 22 forme la branche inspiratoire du circuit patient 29, alors que la ligne de récupération 27 des gaz expirés forme la branche expiratoire du circuit patient 29.

La ligne d'alimentation 22 en gaz est raccordée fluidiquement à un port de sortie 23a du ventilateur médical 23 de manière à récupérer et acheminer le gaz, typiquement de l'air (ou mélange $N_2/O_2$ contenant environ 21% d'$O_2$) délivré par le ventilateur médical 23, alors que la ligne de récupération 27 des gaz expirés est raccordée fluidiquement à un port d'entrée 23b du ventilateur médical 23 de manière à fournir au ventilateur médical 23 tout ou partie du débit des gaz expirés par le patient.

La ligne de récupération 27 des gaz expirés peut comprendre un ou d'autres composants 26 optionnels, comme par exemple un dispositif d'élimination du $CO_2$, i.e. un piège à $CO_2$, tel un bac à chaud ou autre, permettant d'éliminer le $CO_2$ présent dans les gaz expirés par le patient ou un filtre ou autre. En effet, un dispositif d'élimination du $CO_2$ peut être utile lorsque le gaz contient du $N_2O$ à récupérer après son exhalation par le patient. La ligne de récupération 27 des gaz expirés est utilisée, dans le cas du NO, par le ventilateur 23 pour vérifier s'il existe une fuite de gaz dans le circuit 22, 27 par exemple.

Il est aussi prévu un capteur de débit 36, par exemple du type à fil chaud ou à différentiel de pression, relié au dispositif de fourniture de gaz 1 via une ligne de mesure de débit 35 servant à mesurer le débit de gaz (Q') provenant du ventilateur 23, tel de l'air (i.e. $N_2/O_2$), au sein de la ligne d'alimentation 22, en amont du site de raccordement et de mélange $NO/N_2$/air (en 37a). Comme expliqué ci-avant, ceci permet notamment de réguler le passage du NO au travers des électrovannes 5 du dispositif 1.

Par ailleurs, on peut prévoir une ligne de prélèvement 38 de gaz reliant fluidiquement le dispositif de fourniture de gaz 1 à la ligne d'alimentation 22, à proximité de la pièce en Y 28, servant à prélever des échantillons de gaz et à vérifier leur conformité avec le mélange souhaité devant être administré au patient. La ligne de prélèvement 38 est reliée au conduit 22, i.e. la branche inspiratoire du circuit patient 29, en aval (en 38a) du site de liaison 37a du conduit d'injection 37, en considérant le sens de circulation du flux gazeux du ventilateur 23 vers le patient.

Le flux gazeux final (i.e. mélange $NO/N_2/O_2$) résultant du mélange du flux d'air provenant du ventilateur 23 et du flux de NO/N2 provenant de l'appareil 1 est fourni au patient via une interface respiratoire, tel un masque respiratoire ou une sonde d'intubation trachéale, qui sont schématisés ici par un « poumon artificiel » 25.

Fig. 3 schématise un mode de réalisation de l'écran de l'afficheur graphique 7 d'un appareil 1 de fourniture de gaz selon l'invention, lequel est configuré pour afficher différentes informations, comme les concentrations en NO, en $O_2$ et en $NO_2$ (en 14) dans le gaz, mais aussi, selon l'invention une unique touche (e.g. une fenêtre ou analogue) tactile 9a de « Début » et de « Fin » de fourniture de gaz (e.g. NO) permettant à l'utilisateur, i.e. un personnel soignant, de débuter ou stopper temporaire ou définitivement un traitement par administration de gaz, tel du NO, à un patient.

L'écran de l'afficheur graphique 7 affiche, comme expliqué ci-avant, la durée totale $D_{tot}$ (en 10) de fourniture effective de gaz au ou aux patients ayant été traités avec cet appareil 1.

La problématique d'enregistrement du temps de traitement des patients étant globale pour les centres hospitaliers publics et privés, le dispositif de l'invention peut servir à d'autres traitements par gaz que celui par monoxyde d'azote inhalé, i.e. iNO, par exemple pour les traitements à base de mélange équimolaire d'oxygène et de protoxyde d'azote, tel MEOPA, ou un autre gaz ou mélange gazeux (e.g. argon/$O_2$, He/$O_2$...).

Selon un mode de réalisation, les moyens de pilotage 6, en particulier le compteur temporel intégré auxdits moyens de pilotage 6, sont aussi configurés pour déterminer, i.e. mesurer, la durée instantanée ($D_{inst}$) d'un traitement en cours d'un patient spécifique donné, c'est-à-dire le temps total de traitement de ce patient spécifique, en particulier un traitement par NO, et commander un affichage, sur l'afficheur graphique 7, de cette durée instantanée ($D_{inst}$) correspondant au temps de traitement du patient en cours, en particulier lors d'un traitement par administration de NO.

**[0078]** Ce comptage et l'affichage de durée instantanée ($D_{inst}$) relatifs à un 'traitement en cours' sont remis à zéro quand un personnel soignant lance un démarrage d'un nouveau traitement, en cliquant sur une touche de « Début » de traitement ou de "Nouveau patient" faisant office de moyens de remise à zéro (RAZ) ou réinitialisation, c'est-à-dire de touche de réinitialisation, servant à réinitialiser l'affichage de la durée instantanée ($D_{inst}$) sur l'afficheur graphique à la fin du traitement d'un patient donné et avant le début de traitement d'un patient suivant.

Cette durée instantanée ($D_{inst}$) d'un traitement en cours d'un patient spécifique donné peut aussi servir à déterminer si ce traitement est un traitement court TC ou un traitement long TL, et donc le nombre de traitements courts (TC) pour lesquels la durée totale de traitement est inférieure à une durée-seuil préfixée et leurs durées respectives ($D_{TC}$), et/ou, à l'inverse, le nombre de traitements longs (TL) pour lesquels la durée totale de traitement est supérieure à la durée-seuil préfixée et leurs durées respectives ($D_{TL}$), de préférence une durée-seuil préfixée inférieure ou égale à 6 heures. Avantageusement, un traitement court TC a par ailleurs une durée minimale, i.e. seuil-bas, d'au moins 10 min.

D'une façon générale, l'invention porte aussi sur l'utilisation pour traiter un patient souffrant d'une pathologie pulmonaire, d'une installation d'administration de gaz 20 à un patient comprenant une (des) source de gaz 21 contenant un mélange $NO/N_2$, comprenant l'appareil de fourniture 1 de gaz selon l'invention, alimenté en gaz par la (les) source de gaz thérapeutique 21, et un ventilateur médical 23 délivrant un flux gazeux contenant au moins 20% d'oxygène, tel que de l'air ou un mélange $O_2/N_2$, lesquels sont reliés fluidiquement avec une ligne d'alimentation en gaz 22 pour y mélanger le mélange $NO/N_2$ au flux gazeux contenant au moins 20% d'oxygène et obtenir un mélange $NO/N_2/O_2$ contenant principalement du NO, de l'azote et de l'oxygène qui est ensuite administré par inhalation au patient à traiter. Le monoxyde d'azote (NO) présent dans le mélange $NO/N_2/O_2$ inhalé par le patient est un médicament gazeux permettant de traiter l'hypertension artérielle pulmonaire aiguë, en particulier les vasoconstrictions pulmonaires chez l'adulte ou l'enfant, y compris les nouveau-nés, telle que la PPHN ou hypertension pulmonaire persistante du nouveau-né, ou dans le cadre d'une chirurgie cardiaque avec mise en circulation sanguine extracorporelle (CEC) susceptible d'engendrer des vasoconstrictions pulmonaires.

Une telle installation d'administration de NO est principalement destinée à une utilisation en milieu hospitalier pour administrer le traitement par NOi et ainsi soigner les patients de l'hôpital ayant besoin d'inhaler du NO pour traiter leur hypertension artérielle pulmonaire.

**Revendications**

**1.** Appareil de fourniture (1) de gaz comprenant :

- au moins un passage interne (2) pour fournir un flux de gaz, comprenant des moyens à valve (5),
- des moyens de pilotage (6) à microprocesseur commandant les moyens à valve (5) pour contrôler la fourniture de gaz, et
- des moyens de sélection (8), actionnables par un utilisateur, configurés pour permettre à l'utilisateur de débuter ou d'arrêter une fourniture de gaz,

**caractérisé en ce que** les moyens de pilotage (6) sont configurés pour comptabiliser une durée totale ($D_{tot}$) de fourniture effective de gaz par l'appareil (1) pour traiter des patients correspondant au cumul de toutes les périodes de temps (dti) s'étant écoulées entre un début et un arrêt de fourniture de gaz par l'appareil à tous les patients ayant été traités successivement par administration de gaz en utilisant l'appareil, et ce, depuis sa première utilisation ou mise en service.

**2.** Appareil selon la revendication 1, **caractérisé en ce qu'**il comprend un afficheur graphique (7) commandé par les moyens de pilotage (6) et/ou les moyens de sélection (8) comprennent une ou des touches de sélection (9a-9d)

**3.** Appareil selon la revendication 2, **caractérisé en ce que** l'afficheur graphique (7) comprend un écran tactile (7a) et la ou les touches de sélection (9a-9d) sont des touches virtuelles s'affichant sur l'écran tactile (7a).

**4.** Appareil selon l'une des revendications 1 et 2 ou 3, **caractérisé en ce que** les moyens de pilotage (6) sont configurés pour commander un affichage sur l'afficheur graphique (7) de la durée totale ($D_{tot}$) de fourniture effective de gaz.

**5.** &-Appareil selon la revendication 1, **caractérisé en ce que** :

- les moyens de sélection (8) coopèrent avec les moyens de pilotage (6) pour leur fournir au moins un signal de commande correspondant au choix de début ou d'arrêt de fourniture de gaz opéré par l'utilisateur, et
- les moyens de pilotage (6) commandent les moyens à valve (5) pour contrôler le flux de gaz dans ledit au moins un passage interne (2), en réponse au signal de commande fourni par les moyens de sélection (8), de manière à autoriser ou stopper toute circulation de gaz.

**6.** Appareil selon la revendication 1, **caractérisé en ce que** les moyens de pilotage (6) comprennent un compteur temporel intégré aux moyens de pilotage (6), en particulier au microprocesseur, en particulier un algorithme faisant office de compteur temporel.

**7.** Appareil selon la revendication 16, **caractérisé en ce que** le compteur temporel intégré aux moyens de pilotage (6) est configuré pour comptabiliser toutes les périodes de temps (dti) s'écoulant entre un choix de début de fourniture de gaz (DF) et un choix d'arrêt de fourniture de gaz (AF) opérés par l'utilisateur, en excluant toute période de non-fourniture de gaz (PA, IP).

**8.** Appareil selon la revendication 1, **caractérisé en ce qu'**il comprend des moyens de mémorisation pour mémoriser la durée totale ($D_{tot}$) de fourniture effective de gaz et/ou toutes les périodes de temps (dti).

**9.** Appareil selon les revendications 1 et 2. **caractérisé en ce que** les moyens de pilotage (6) sont par ailleurs configurés pour déterminer une durée instantanée ($D_{inst}$) d'un traitement en cours et l'afficheur graphique (7) est configuré pour afficher ladite durée instantanée ($D_{inst}$).

**10.** Appareil selon les revendications 1 et 9, **caractérisé en ce que** les moyens de pilotage (6) sont configurés pour déterminer un traitement court (TC) ou un traitement long (TL) à partir de la durée instantanée ($D_{inst}$) d'un traitement en cours d'un patient donné.

**11.** Installation d'administration de gaz (20) à un patient comprenant :

- au moins une source de gaz (21), tel un mélange $NO/N_2$,
- un appareil de fourniture (1) de gaz selon l'une des revendications précédentes, alimenté en gaz par ladite au moins une source de gaz thérapeutique (21), et
- une ligne d'alimentation en gaz (22) alimentée en gaz par l'appareil de fourniture (1) de gaz.

**12.** Installation selon la revendication 11, **caractérisé en ce qu'**elle comporte en outre un ventilateur médical (23) en communication fluidique avec la ligne d'alimentation en gaz (22).

**13.** Installation selon la revendication 11, **caractérisé en ce que** ladite au moins une source de gaz (21) contient un mélange **$NO/N_2$.**

**14.** Installation selon la revendication 11, **caractérisé en ce que** la source de gaz contient un mélange $NO/N_2$ contenant moins de 2000 ppm en volume de NO, le reste étant de l'azote, de préférence moins de 1000 ppm en volume de NO, le reste étant de l'azote.

**Patentansprüche**

**1.** Eine Gaszuführungsvorrichtung (1), umfassend:

- mindestens einen inneren Durchgang (2) zum Zuführen eines Gasstroms, der Ventilmittel (5) umfasst,
- Steuerungsmittel (6) mit Mikroprozessor, die die Ventilmittel (5) zur Steuerung der Gaszufuhr befehligen, und
- Auswahlmittel (8), die von einem Benutzer betätigbar sind und so konfiguriert sind, dass sie es dem Benutzer ermöglichen, eine Gaszufuhr zu starten oder zu stoppen,

**dadurch gekennzeichnet, dass** die Steuerungsmittel (6) so konfiguriert sind, dass sie eine Gesamtdauer (Dtot) der effektiven Gaszufuhr durch die Vorrichtung (1) zur Behandlung von Patienten erfassen, die der Summe aller Zeiträume (dti) entspricht, die zwischen einem Beginn und einem Ende der Gaszufuhr durch die Vorrichtung zu allen Patienten vergangen sind, die nacheinander durch Gasverabreichung unter Verwendung der Vorrichtung behandelt wurden, und dies seit ihrer ersten Verwendung oder Inbetriebnahme.

**2.** Vorrichtung nach Anspruch 1, **dadurch gekennzeichnet, dass** sie eine grafische Anzeige (7) umfasst, die von den Steuerungsmitteln (6) befehligt wird und/oder die Auswahlmittel (8) eine oder mehrere Auswahltasten (9a-9d) umfassen.

**3.** Vorrichtung nach Anspruch 2, **dadurch gekennzeichnet, dass** die grafische Anzeige (7) einen Touchscreen (7a) umfasst und die eine oder die mehreren Auswahltasten (9a-9d) virtuelle Tasten sind, die auf dem Touchscreen (7a) angezeigt werden.

**4.** Vorrichtung nach einem der Ansprüche 1 und 2 oder 3, **dadurch gekennzeichnet, dass** die Steuerungsmittel (6) so konfiguriert sind, dass sie eine Anzeige der Gesamtdauer (Dtot) der effektiven Gaszufuhr auf der grafischen Anzeige (7) befehligen.

**5.** Vorrichtung nach Anspruch 1, **dadurch gekennzeichnet, dass**:

- die Auswahlmittel (8) mit den Steuerungsmitteln (6) zusammenwirken, um ihnen mindestens ein Befehlssignal zu liefern, das der vom Benutzer getroffenen Wahl des Beginns oder des Endes der Gaszufuhr entspricht, und
- die Steuerungsmittel (6) die Ventilmittel (5) zur Steuerung des Gasflusses in dem besagten mindestens einen inneren Durchgang (2) als Reaktion auf das von den Auswahlmitteln (8)

gelieferte Befehlssignal befehlen, um jegliche Gaszirkulation zu genehmigen oder zu stoppen.

6. Vorrichtung nach Anspruch 1, **dadurch gekennzeichnet, dass** die Steuerungsmittel (6) einen in die Steuerungsmittel (6) integrierten Zeitzähler umfassen, insbesondere in den Mikroprozessor, insbesondere einen Algorithmus, der als Zeitzähler fungiert.

7. Vorrichtung nach Anspruch 6, **dadurch gekennzeichnet, dass** der in die Steuerungsmittel (6) integrierte Zeitzähler so konfiguriert ist, dass er alle Zeiträume (dti) erfasst, die zwischen einer Wahl des Beginns der Gaszufuhr (DF) und einer Wahl des Endes der Gaszufuhr (AF), die vom Benutzer vorgenommen wurden, verstreichen, unter Ausschluss jeglichen Zeitraums der Nicht-Gaszufuhr (PA, IP).

8. Vorrichtung nach Anspruch 1, **dadurch gekennzeichnet, dass** sie Speichermittel zum Speichern der Gesamtdauer (Dtot) der effektiven Gaszufuhr und/oder aller Zeiträume (dti) umfasst.

9. Vorrichtung nach den Ansprüchen 1 und 2, **dadurch gekennzeichnet, dass** die Steuerungsmittel (6) außerdem so konfiguriert sind, dass sie eine momentane Dauer (Dinst) einer laufenden Behandlung bestimmen, und die grafische Anzeige (7) so konfiguriert ist, dass sie die besagte momentane Dauer (Dinst) anzeigt.

10. Vorrichtung nach den Ansprüchen 1 und 9, **dadurch gekennzeichnet, dass** die Steuerungsmittel (6) so konfiguriert sind, dass sie eine Kurzzeitbehandlung (TC) oder eine Langzeitbehandlung (TL) aus der momentanen Dauer (Dinst) einer laufenden Behandlung eines bestimmten Patienten bestimmen.

11. Eine Gasverabreichungsanlage (20) an einen Patienten, umfassend:

- mindestens eine Gasquelle (21), wie z. B. ein NO/N2-Gemisch,
- eine Gaszuführungsvorrichtung (1) nach einem der vorhergehenden Ansprüche, die mit Gas von der besagten mindestens einen therapeutischen Gasquelle (21) versorgt wird, und
- eine Gaszufuhrleitung (22), die mit Gas von der Gaszuführungsvorrichtung (1) versorgt wird.

12. Anlage nach Anspruch 11, **dadurch gekennzeichnet, dass** sie ferner ein medizinisches Beatmungsgerät (23) in fluider Kommunikation mit der Gaszufuhrleitung (22) aufweist.

13. Anlage nach Anspruch 11, **dadurch gekennzeichnet, dass** die besagte mindestens eine Gasquelle (21) ein NO/N2-Gemisch enthält.

14. Anlage nach Anspruch 11, **dadurch gekennzeichnet, dass** die Gasquelle ein NO/N2-Gemisch enthält, das weniger als 2000 ppm Volumenanteil an NO enthält, wobei der Rest Stickstoff ist, vorzugsweise weniger als 1000 ppm Volumenanteil an NO, wobei der Rest Stickstoff ist.

## Claims

1. A gas supply apparatus (1) comprising:

- at least one internal passage (2) for supplying a gas flow, comprising valve means (5),
- microprocessor-based control means (6) for commanding the valve means (5) to control the gas supply, and
- selection means (8), actuable by a user, configured to allow the user to start or stop a gas supply,

**characterized in that** the control means (6) are configured to account for a total duration (Dtot) of effective gas supply by the apparatus (1) for treating patients corresponding to the cumulation of all time periods (dti) elapsed between a start and a stop of gas supply by the apparatus to all patients having been successively treated by gas administration using the apparatus, and this, since its first use or commissioning.

2. The apparatus according to claim 1, **characterized in that** it comprises a graphic display (7) controlled by the control means (6) and/or the selection means (8) comprise one or more selection keys (9a-9d).

3. The apparatus according to claim 2, **characterized in that** the graphic display (7) comprises a touchscreen (7a) and the one or more selection keys (9a-9d) are virtual keys displayed on the touchscreen (7a).

4. The apparatus according to any one of claims 1 and 2 or 3, **characterized in that** the control means (6) are configured to command a display on the graphic display (7) of the total duration (Dtot) of effective gas supply.

5. The apparatus according to claim 1, **characterized in that**:

- the selection means (8) cooperate with the control means (6) to provide them with at least one command signal corresponding to the choice of starting or stopping the gas supply made by the user, and
- the control means (6) command the valve means (5) to control the gas flow in said at least one internal passage (2), in response to the command signal provided by the selection means (8), so as to authorize or stop any gas circulation.

6. The apparatus according to claim 1, **characterized in that** the control means (6) comprise a time counter integrated into the control means (6), in particular into the microprocessor, in particular an algorithm acting as a time counter.

7. The apparatus according to claim 6, **characterized in that** the time counter integrated into the control means (6) is configured to account for all time periods (dti) elapsing between a choice to start gas supply (DF) and a choice to stop gas supply (AF) made by the user, excluding any period of non-supply of gas (PA, IP).

8. The apparatus according to claim 1, **characterized in that** it comprises memory means for memorizing the total duration (Dtot) of effective gas supply and/or all the time periods (dti).

9. The apparatus according to claims 1 and 2, **characterized in that** the control means (6) are furthermore configured to determine an instantaneous duration (Dinst) of an ongoing treatment and the graphic display (7) is configured to display said instantaneous duration (Dinst).

10. The apparatus according to claims 1 and 9, **characterized in that** the control means (6) are configured to determine a short treatment (TC) or a long treatment (TL) from the instantaneous duration (Dinst) of an ongoing treatment of a given patient.

11. A gas administration installation (20) for a patient comprising:

    - at least one gas source (21), such as an NO/N2 mixture,
    - a gas supply apparatus (1) according to any one of the preceding claims, supplied with gas by said at least one therapeutic gas source (21), and
    - a gas supply line (22) supplied with gas by the gas supply apparatus (1).

12. The installation according to claim 11, **characterized in that** it further comprises a medical ventilator (23) in fluid communication with the gas supply line (22).

13. The installation according to claim 11, **characterized in that** said at least one gas source (21) contains an NO/N2 mixture.

14. The installation according to claim 11, **characterized in that** the gas source contains an $NO/N_2$ mixture containing less than 2000 ppm by volume of NO, the remainder being nitrogen, preferably less than 1000 ppm by volume of NO, the remainder being nitrogen.

Fig. 1

Fig. 2

Fig. 3

Fig. 4

$$D_{tot} = dt1 + dt2 + dt3 + \ldots\ldots$$

**RÉFÉRENCES CITÉES DANS LA DESCRIPTION**

**Documents brevets cités dans la description**

- EP 560928 A
- EP 1516639 A
- EP 3821929 A